# EUROPEAN PATENT SPECIFICATION

(11) **EP 4 341 430 B1**
(45) Date of publication and mention of the grant of the patent: **08.07.2026**
(21) Application number: 22805604.0
(22) Date of filing: 20.05.2022
(51) Int. Cl.: C12Q 1/6844

(54) **UNIVERSAL LAMP ASSAYS FOR DETECTION OF NUCLEIC ACID TARGETS**
UNIVERSELLE LAMPENTESTS ZUM NACHWEIS VON NUKLEINSÄUREZIELMOLEKÜLEN
DOSAGES UNIVERSELS DE LAMP POUR LA DÉTECTION DE CIBLES D'ACIDES NUCLÉIQUES

(30) Priority: 21.05.2021 US 202163191590 P
(43) Date of publication of application: 27.03.2024
(73) Proprietor: Duke University, Durham, NC 27705 (US)
(72) Inventor: REIF, John H., Durham, NC 27707 (US); SONG, Xin, Raleigh, NC 27606 (US)
(74) Representative: Schwarz & Partner Patentanwälte GmbH
(86) International application number: PCT/US2022/030312
(87) International publication number: WO 2022/246237

(56) References cited:
- WO-A1-2020/227444
- NOTOMI TSUGUNORI ET AL: "Loop-mediated isothermal amplification (LAMP): principle, features, and future prospects", THE JOURNAL OF MICROBIOLOGY, THE MICROBIOLOGICAL SOCIETY OF KOREA // HAN-GUG MISAENGMUL HAG-HOE, KR, vol. 53, no. 1, 4 January 2015 (2015-01-04), pages 1 - 5, XP035419145, ISSN: 1225-8873, [retrieved on 20150104], DOI: 10.1007/S12275-015-4656-9
- RABE BRIAN A., CEPKO CONSTANCE: "SARS-CoV-2 detection using isothermal amplification and a rapid, inexpensive protocol for sample inactivation and purification", PROCEEDINGS OF THE NATIONAL ACADEMY OF SCIENCES, NATIONAL ACADEMY OF SCIENCES, vol. 117, no. 39, 29 September 2020 (2020-09-29), pages 24450 - 24458, XP055910088, ISSN: 0027-8424, DOI: 10.1073/pnas.2011221117
- MIN HAO, JIANJUN QIAO, HAO QI: "Current and Emerging Methods for the Synthesis of Single-Stranded DNA", GENES, vol. 11, no. 2, 2020, pages 116, XP055712647, DOI: 10.3390/genes11020116
- CYRILL, S.L. ET AL.: "Universal Template-Assisted, Cloning-free Method for the Generation of Small RNA-Expressing Dumbbell-Shaped DNA Vectors", MOLECULAR THERAPY METHODS & CLINICAL DEVELOPMENT, vol. 15, 13 December 2019 (2019-12-13), pages 149 - 156, XP055700403, DOI: 10.1016/j.omtm. 2019.08.00 8

## Description

### CROSS-REFERENCE TO RELATED APPLICATIONS

This application claims the benefit of and priority to United States Provisional Patent Application No. 63/191,590, filed on May 21, 2021.

### BACKGROUND

Loop-mediated isothermal amplification (LAMP) and reverse-transcription LAMP (RT-LAMP) (see Tomita, N.; Mori, Y.; Kanda, H.; Notomi, T. Loop-Mediated Isothermal Amplification (LAMP) of Gene Sequences and Simple Visual Detection of Products. Nat. Protoc. 2008, 3, 877-882 and Parida, M.; Sannarangaiah, S.; Dash, P. K.; Rao, P. V. L.; Morita, K. Loop Mediated Isothermal Amplification (LAMP): A New Generation of Innovative Gene Amplification Technique; Perspectives in Clinical Diagnosis of Infectious Diseases. Rev. Med. Virol. 2008, 18, 407-42: and Notomi, T.; Mori, Y.; Tomita, N.; Kanda, H. Loop-mediated isothermal amplification (LAMP): principle, features, and future prospects J Microbiol 2015, 53(1), 1-5) are powerful molecular techniques for rapid exponential amplification of nucleic acids under isothermal conditions without the need for expensive thermal cycling equipment and time-consuming protocols as required in PCR and RT-PCR assays (see Zhao, Y.; Chen, F.; Li, Q.; Wang, L.; Fan, C. Isothermal Amplification of Nucleic Acids. Chem. Rev. 2015, 115, 12491-12545).

Advantages such as simple reaction setup, short turnaround time, multiple available readout methods, high tolerance to common inhibitors, and possibility to bypass nucleic acid extraction make LAMP/RT-LAMP-based diagnostics especially attractive for inexpensive point-of-care (POC) testing and near-patient testing of emerging pathogens (see Wong, Y.-P.; Othman, S.; Lau, Y.-L.; Radu, S.; Chee, H.-Y. Loop-Mediated Isothermal Amplification (LAMP): A Versatile Technique for Detection of Micro-Organisms. J. Appl. Microbiol. 2018, 124, 626-643) and infectious diseases (see Mori, Y.; Notomi, T. Loop-Mediated Isothermal Amplification (LAMP): A Rapid, Accurate, and Cost-Effective Diagnostic Method for Infectious Diseases. J. Infect. Chemother. 2009, 15, 62-69).

However, the high sensitivity and specificity of LAMP/RT-LAMP reaction fundamentally depends on the meticulous design and optimization of a set of six primers which recognize eight distinct regions on the target sequence (see Moehling, T. J.; Choi, G.; Dugan, L. C.; Salit, M.; Meagher, R. J. LAMP Diagnostics at the Point-of-Care: Emerging Trends and Perspectives for the Developer Community. Expert Rev. Mol. Diagn. 2021, 21, 43-61). The necessity to completely redesign, screen, and optimize new sets of LAMP primers for each desired target makes it costly, time-consuming, and labor-intensive to develop high-performance LAMP/RT-LAMP assays in response to evolving pathogens and emerging infectious diseases.

Furthermore, to detect multiple targets, most state-of-the-art methods of multiplexed LAMP/RT-LAMP must rely on the inclusion of multiple orthogonal LAMP primer sets in the reaction mix (see Zhang, Y.; Tanner, N. A. Development of Multiplexed Reverse-Transcription Loop-Mediated Isothermal Amplification for Detection of SARS-CoV-2 and Influenza Viral RNA. Biotechniques 2021, 70, 167-174 and Jang, W. S.; Lim, D. H.; Yoon, J.; Kim, A.; Lim, M.; Nam, J.; Yanagihara, R.; Ryu, S.-W.; Jung, B. K.; Ryoo, N.-H.; et al. Development of a Multiplex Loop-Mediated Isothermal Amplification (LAMP) Assay for on-Site Diagnosis of SARS CoV-2. PLoS One 2021, 16, e0248042) as well as special readout mechanisms such as multiple fluorophore-quencher pairs (see Tanner, N. A.; Zhang, Y.; Evans, T. C. Simultaneous Multiple Target Detection in Real-Time Loop-Mediated Isothermal Amplification. Biotechniques 2012, 53, 81-89), microcapillaries (see Zhang, Y.; Zhang, L.; Sun, J.; Liu, Y.; Ma, X.; Cui, S.; Ma, L.; Xi, J. J.; Jiang, X. Point-of-Care Multiplexed Assays of Nucleic Acids Using Microcapillary-Based Loop-Mediated Isothermal Amplification. Anal. Chem. 2014, 86, 7057-7062 and Li, R.; Chen, J.; Zhang, X.; Cui, J.; Tao, S.; Yang, L. Mini-Disk Capillary Array Coupling with LAMP for Visual Detection of Multiple Nucleic Acids Using Genetically Modified Organism Analysis as an Example. J. Agric. Food Chem. 2020, 68, 899-906), lateral flow devices (see Zhu, X.; Wang, X.; Han, L.; Chen, T.; Wang, L.; Li, H.; Li, S.; He, L.; Fu, X.; Chen, S.; et al. Multiplex Reverse Transcription Loop-Mediated Isothermal Amplification Combined with Nanoparticle-Based Lateral Flow Biosensor for the Diagnosis of COVID-19. Biosens. Bioelectron. 2020, 166, 112437), or amplicon melting patterns (see Dong, J.; Xu, Q.; Li, C.; Zhang, C. Single-Color Multiplexing by the Integration of High-Resolution Melting Pattern Recognition with Loop-Mediated Isothermal Amplification. Chem. Commun. 2019, 55, 2457-2460) to interpret the reaction results. These implementations significantly increase the overall cost and complexity of the assays and may also lead to a higher risk of contamination (e.g., if the reaction vessel must be reopened post amplification for readout and analysis).

To simplify the implementation of multiplexed LAMP, a recent work proposed the idea of mediator-reporter set (see Becherer, L.; Bakheit, M.; Frischmann, S.; Stinco, S.; Borst, N.; Zengerle, R.; von Stetten, F. Simplified Real-Time Multiplex Detection of Loop-Mediated Isothermal Amplification Using Novel Mediator Displacement Probes with Universal Reporters. Anal. Chem. 2018, 90, 4741-4748), which enables the use of a single florescent hairpin reporter to visualize the amplification products generated from different LAMP reactions. While this prior design provides an efficient readout method for multiplexed LAMP, it still does not bypass the requirement to meticulously design, screen, and optimize a full set of LAMP primers for every single target involved in the assay.

Accordingly, there is an ongoing need for improved systems, methods, and associated workflows for LAMP/RT-LAMP, particularly for use in assays for responding to evolving pathogens and emerging infectious diseases.

### SUMMARY

The present disclosure is directed to universal LAMP/RT-LAMP assays for effective detection of distinct DNA/RNA targets. In conventional LAMP/RT-LAMP reactions, a set of six unique primers must be meticulously designed and optimized for each desired target sequence. Due to numerous design constraints on LAMP primers, the design and optimization of LAMP/RT-LAMP assays are usually difficult and require tedious empirical testing of multiple candidate primer sets for a desired target. As a result, the detection of additional targets requires the complete re-design, screening, and optimization of new sets of LAMP primers, which can be a very costly, time-consuming, and labor-intensive process.

The present disclosure integrates a novel transduction mechanism that functions upstream of a highly optimized LAMP reaction to effectively transduce the detection of any target DNA/RNA sequence into the rapid amplification of a universal LAMP template that is independent of the assay's target sequence. The transduction mechanism (Figure 1) comprises the target sequence and three easy-to-design primers that undergo simple hybridization and strand-displacing polymerization reactions to release one or multiple copies of a universal ssDNA oligo (Figure 2), whose sequence is pre-designed to function as one of the essential LAMP primers to initiate a downstream universal LAMP reaction (Figure 3, Figure 4).

This LAMP reaction is highly optimized and does not need to be readjusted for detection of different targets. As a result, the present invention enables rapid development of universal one-pot molecular assays using just a single set of highly optimized LAMP primers to detect any desired set of distinct DNA or RNA sequences, with high specificity and sensitivity. The simplicity of the universal assay design facilitates fast deployment of inexpensive and accurate molecular diagnostics to enable widespread point-of-care/near-patient testing of current/emerging pathogens (e.g., viruses, bacteria, fungi), including infectious diseases of global public health importance such as Ebola, Zika, SARS-CoV-2, malaria, tuberculosis, dengue, yellow fever, etcetera. Other applications include analysis of environmental samples. For example, certain embodiments may be directed to environmental monitoring and assessment by analyzing water samples, soil samples, and/or other types of environmental samples.

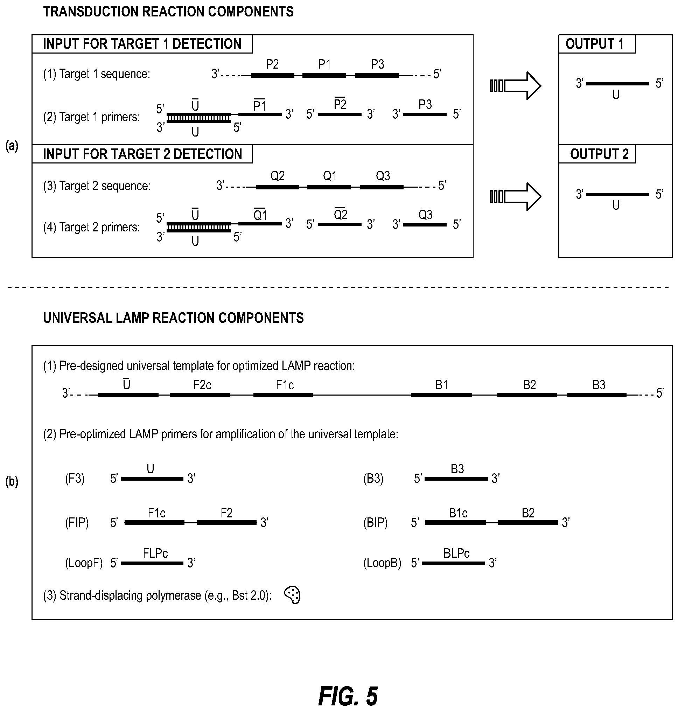

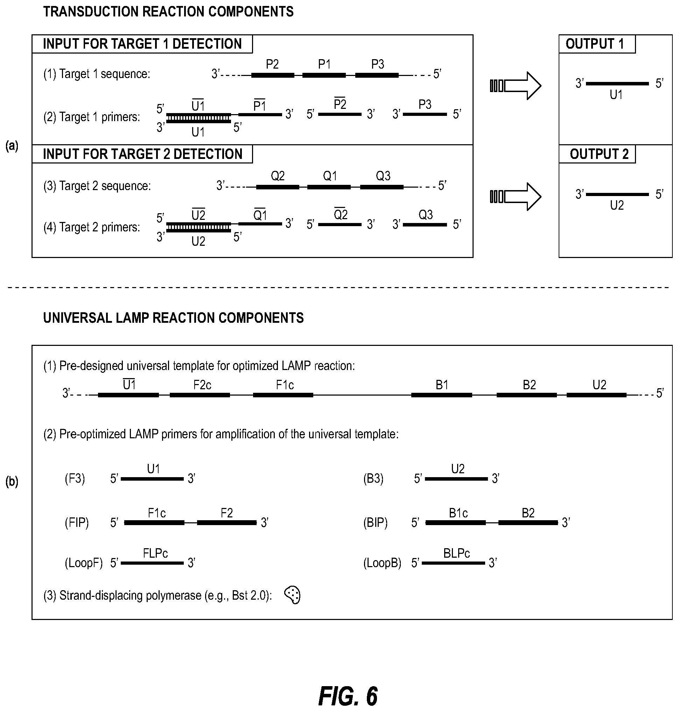

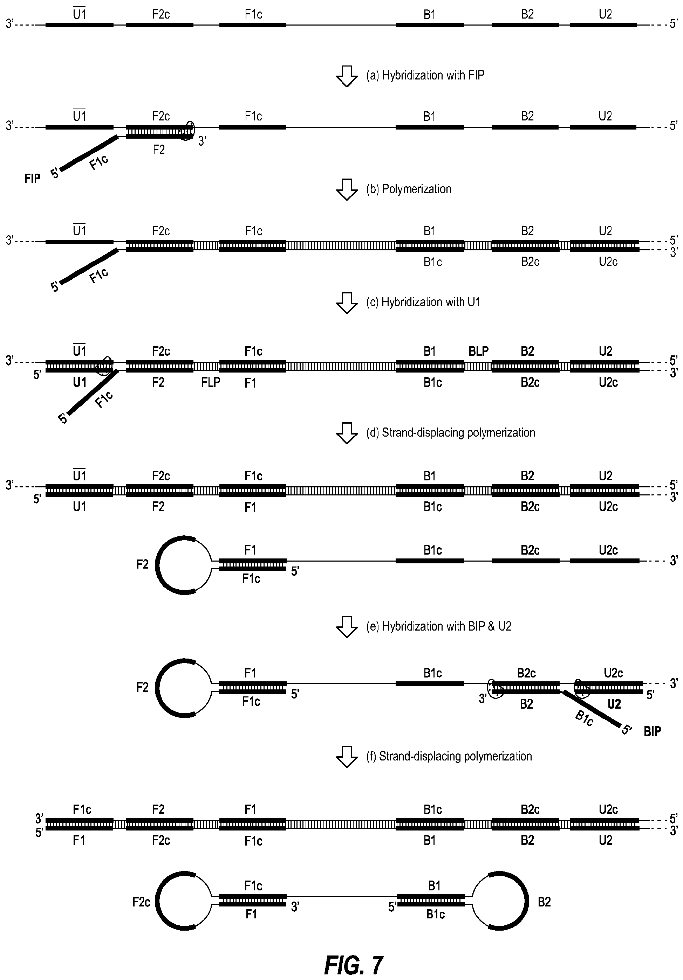

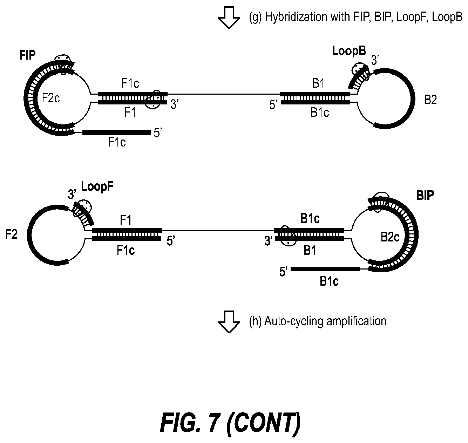

Furthermore, the presently disclosed embodiments relate to designs and implementations of multiplexed universal LAMP/RT-LAMP assays with direct support for Boolean 'Logic OR' (Figure 5) and 'Logic AND' (Figure 6, Figure 7) computation in detection of multiple targets, enabling easy customization and enhancement of the assay sensitivity, specificity, and degree of multiplexity. The presently disclosed embodiments achieve universal rapid isothermal detection of nucleic acids in either singleplex or multiplex assay formats, offering higher sensitivity and specificity than PCR/RT-PCR while featuring simplified assay designs and lower development cost than conventional multiplexed LAMP/RT-LAMP.

The novel transduction mechanism as described herein is broadly compatible with various isothermal amplification methods and readout techniques. In addition to molecular diagnostic applications, the proposed transduction mechanism can be broadly applied to the field of DNA and RNA computing to achieve simple universal transduction of input and output signals in complex molecular circuitries.

This summary is provided to introduce a selection of concepts in a simplified form that are further described below in the detailed description. This summary is not intended to identify key features or essential features of the claimed subject matter, nor is it intended to be used as an indication of the scope of the claimed subject matter.

### BRIEF DESCRIPTION OF THE DRAWINGS

Various objects, features, characteristics, and advantages of the invention will become apparent and more readily appreciated from the following description of the embodiments, taken in conjunction with the accompanying drawings and the appended claims, all of which form a part of this specification. In the Drawings, like reference numerals may be utilized to designate corresponding or similar parts in the various Figures, and the various elements depicted are not necessarily drawn to scale, wherein:

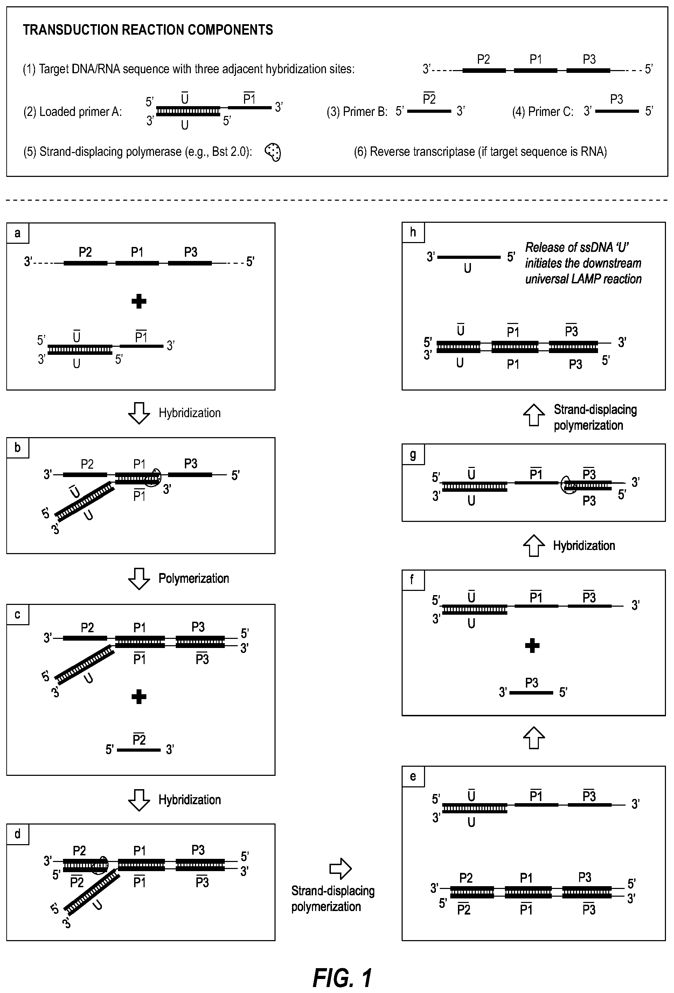

Figure 1 illustrates an exemplary transduction reaction showing hybridization and strand-displacing polymerization reactions between the target sequence and three simple-to-design primers resulting in the release of an ssDNA oligo 'U' that functions as one of the LAMP primers for a downstream pre-designed optimized universal LAMP reaction;

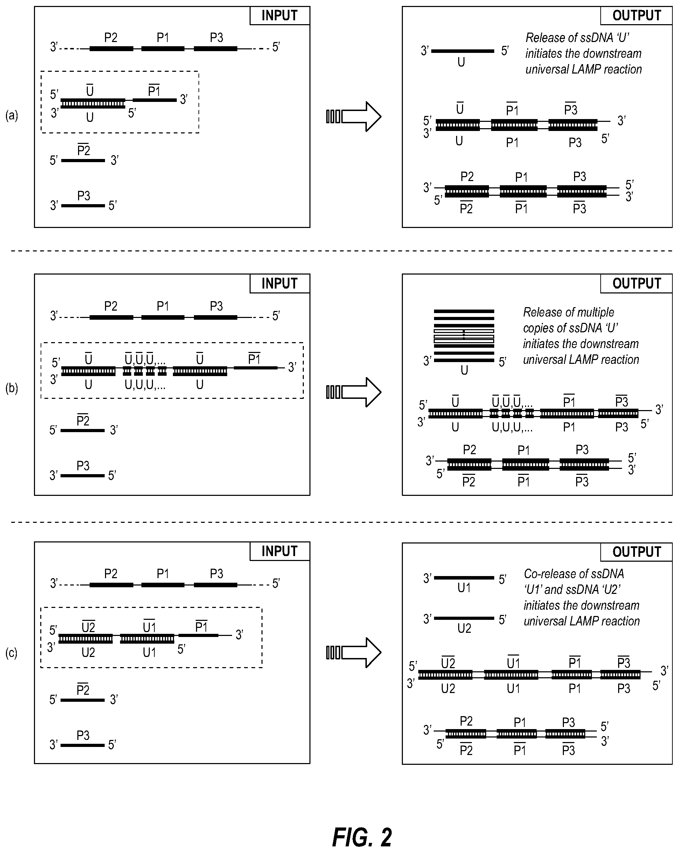

Figure 2 illustrates design schemes of the loaded primer for the transduction reaction, showing in the second design scheme that the loaded primer includes repeated domains that are each hybridized to a copy of 'U', allowing the transduction reaction to quickly release multiple copies of 'U' upon detection of a single copy of the target sequence, and showing that the backbone for the loaded primer with repeated domains can be easily prepared by direct synthesis or using techniques such as primer exchange reactions, and showing in the third design scheme the output of the transduction reaction is the co-release of 'U1' and 'U2', which function as two of the six primers for the downstream universal LAMP reaction;

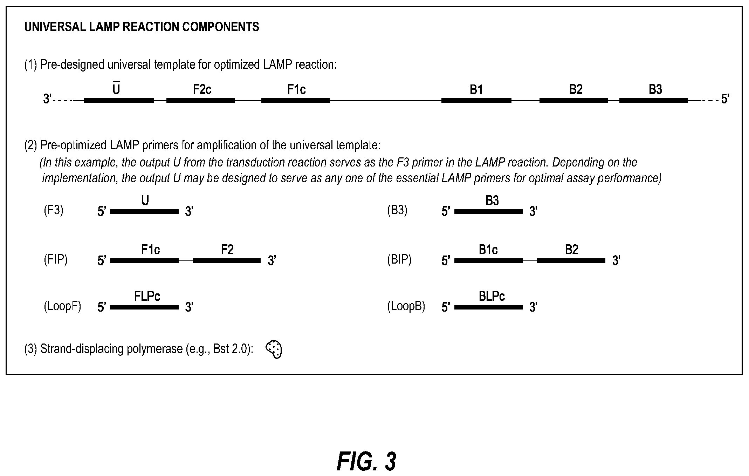

Figure 3 illustrates universal LAMP reaction components;

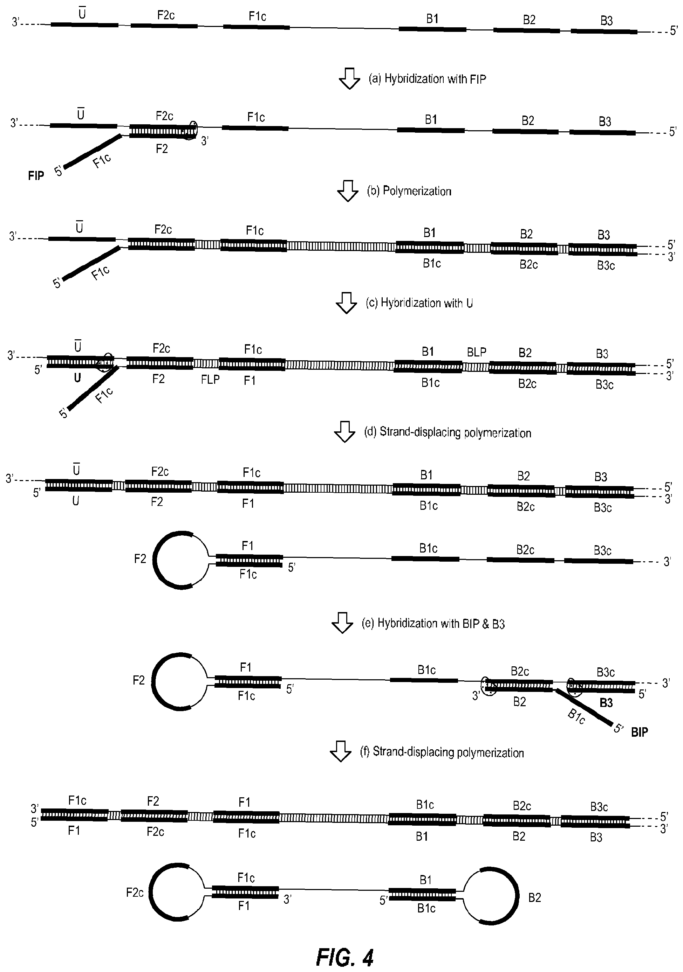

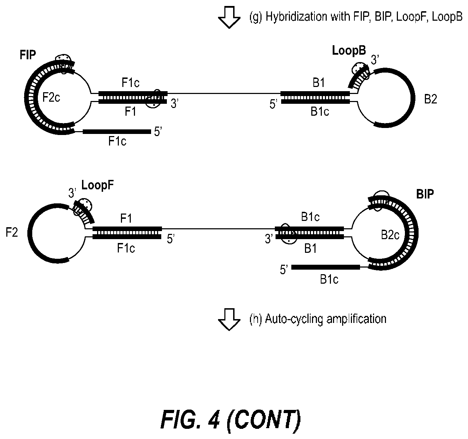

Figure 4 illustrates an example of a universal LAMP reaction, showing that the ssDNA 'U' released from the transduction reaction triggers the rapid initialization of a pre-designed highly optimized LAMP reaction;
Figure 5 illustrates reaction components of multiplexed universal LAMP with 'Logic OR' operation on targets, wherein the reaction steps for universal LAMP with 'Logic OR' operation on targets can be the same as shown in Figure 4;
Figure 6 illustrates reaction components of multiplexed universal LAMP with 'Logic AND' operation on targets; and
Figure 7 illustrates a universal LAMP reaction with 'Logic AND' operation on targets, showing that the co-release of 'U1' and 'U2' from the transduction reaction triggers the initialization of a pre-designed optimized LAMP reaction.

### DETAILED DESCRIPTION

### Overview of Universal LAMP/RT-LAMP Assays

To substantially simplify the design and implementation of LAMP-based assays for effective detection of different targets, the present disclosure describes a simple scheme of universal LAMP/RT-LAMP, which leverages a novel transduction mechanism including three target-specific primers to rapidly initialize a highly optimized universal LAMP reaction upon the detection of any desired set of target DNA or RNA sequences. The coupling between the transduction reaction and the LAMP reaction is the target-triggered release of a universal ssDNA oligonucleotide that functions as one of the essential LAMP primers for a pre-designed, highly optimized LAMP reaction.

Distinct target nucleic acids can therefore be rapidly amplified with high specificity and sensitivity based on the design of only three primers as opposed to, in standard approaches, six specific LAMP primers for every target. In contrast to the state-of-the-art molecular assays that integrate multi-stage reactions with LAMP (see, e.g., Marciniak, J. Y.; Kummel, A. C.; Esener, S. C.; Heller, M. J.; Messmer, B. T. Coupled Rolling Circle Amplification Loop-Mediated Amplification for Rapid Detection of Short DNA Sequences. Biotechniques 2008, 45, 275-280), the presently described assays do not require additional enzymes or increases to the reaction time to achieve a high level of sensitivity and specificity on par with highly optimized LAMP/RT-LAMP assays.

The integration of the simple transduction mechanism with the universal LAMP reaction also facilitates direct implementations of Boolean logic operations during rapid detection of multiple nucleic acid targets. Various alternative design schemes of the 'loaded primer' are further described to enable flexible fine-tuning of the sensitivity, specificity, and multiplexity of the universal LAMP/RT-LAMP assays. Built-in support for Boolean logic computation also allows the use of a simple pH-based colorimetric readout (such as described in Tanner, N. A.; Zhang, Y.; Evans, T. C. Visual Detection of Isothermal Nucleic Acid Amplification Using PH-Sensitive Dyes. Biotechniques 2015, 58, 59-68, for example) or other relatively simple detection scheme for easy interpretation of the multiplexed test result by direct visual inspection.

A multiplexed assay with inherent support for Boolean logic computation offers numerous advantages for practical applications. For example, the multiplexed 'Logic OR' operation can be leveraged to improve the assay multiplexity (e.g., by simultaneously detecting multiple pathogens or multiple subtypes/mutants of a particular pathogen) or to enhance the assay sensitivity (e.g., by simultaneously detecting multiple genomic loci of a particular target pathogen). Similarly, the 'Logic AND' operation can be leveraged to improve the assay multiplexity (e.g., by detecting the co-presence of multiple pathogens or multiple subtypes/mutants of a particular pathogen) or to enhance the assay specificity (e.g., by detecting the co-presence of multiple genomic loci specific to a particular target pathogen).

The presently described assay enables universal rapid isothermal detection of nucleic acids (including but are not limited to DNA, ssDNA, dsDNA, RNA, mRNA, tRNA, microRNA, rRNA, siRNA, sgRNA, and nucleic acid analogues such as TNA, LNA, HNA, GNA) in either singleplex or multiplex assay format, offering higher sensitivity and specificity than PCR/RT-PCR while featuring simplified assay designs and lower development cost than conventional multiplex LAMP/RT-LAMP.

The universal assays as presently described are compatible with multiple readout methods, including but not limited to, colorimetric, fluorescence, chemiluminescence, electrochemical, turbidity, and the like. The presently described assays may also be employed in conjunction with readout mechanisms such as multiple fluorophore-quencher pairs, microcapillaries, lateral flow devices, or amplicon melting patterns.

In addition to applications in molecular diagnostics, the novel transduction mechanism proposed herein can be broadly applied to the field of DNA and RNA computing to easily enable universal transduction of input and output signals in complex molecular circuitries. For examples of such systems where the embodiments described herein may be utilized, see Song, X.; Reif, J. Nucleic Acid Databases and Molecular-Scale Computing. ACS Nano 2019, 13, 6256-6268 and Shah, S.; Wee, J.; Song, T.; Ceze, L.; Strauss, K.; Chen, Y.-J.; Reif, J. Using Strand Displacing Polymerase To Program Chemical Reaction Networks. J. Am. Chem. Soc. 2020, 142, jacs.0c02240.

It will be understood that the universal transduction mechanism proposed herein is also broadly compatible with various other isothermal amplification techniques in addition to LAMP, including but not limited to, strand displacement amplification (SDA), nucleic acid sequence-based amplification (NASBA), rolling cycle amplification (RCA), isothermal multiple displacement amplification (IMDA), helicase-dependent amplification (HDA), recombinase polymerase amplification (RPA), nicking-enzyme amplification reaction (NEAR), single primer isothermal amplification (SPIA), and other variations of exponential amplification, linear amplification, and cascade amplification. See Zhao et al., Chem Rev. 2015 and Gill, P.; Ghaemi, A. Nucleic Acid Isothermal Amplification Technologies-A Review. Nucleosides, Nucleotides and Nucleic Acids 2008, 27, 224-243 for additional discussion of these techniques.

### Example Embodiments

Figure 1 illustrates an example transduction reaction. The purpose of the transduction is to detect the presence of a target nucleic acid strand, and in response, release a universal ssDNA oligo 'U' that functions as one of the essential LAMP primers to rapidly initialize a downstream pre-designed highly optimized universal LAMP reaction.

The illustrated transduction reaction includes these components: (1) a target strand that contains subsequences P2, P1, P3 (listed from 3' to 5' orientation) as three adjacent primer binding sites; (2) a loaded primer "A", that includes a primer nucleic acid strand with subsequences U̅ and P̅1̅ (listed from 5' to 3' orientation), loaded with a nucleic acid strand U hybridized to the subsequence U̅ (the bar notation indicates sequence reverse complementarity); (3) a primer "B", that includes a primer nucleic acid strand P̅2̅; (4) a primer "C" that includes a primer nucleic acid strand P3; (5) a strand-displacing polymerase (e.g., Bst 2.0); and (6) reverse transcriptase (if target sequence is RNA). For brevity, common reagents such as reaction buffer and nuclease-free water are not depicted in the illustration.

Figure 1 also illustrates the hybridization and strand-displacing polymerization reactions between the target strand and the three primers A, B, and C, which result in the release of an ssDNA oligo 'U' that functions as one of the essential LAMP primers for a downstream pre-designed highly optimized universal LAMP reaction.

In particular, step (a) includes the hybridization reaction between the target strand and the loaded primer "A" via the primer binding site P1 on the target strand. Step (b) includes the polymerization reaction initialized from the 3' end of P̅1̅ of the nucleic acid complex produced from step (a). Step (c) includes the hybridization reaction between the primer "B" and the nucleic acid complex produced from step (b) via the primer binding site P2 on the target strand. Step (d) includes the strand-displacing polymerization reaction initialized from the 3' end of P̅2̅ of the nucleic acid complex produced from step (c). Step (e) illustrates the resultant nucleic complexes produced from step (d). Step (f) includes the hybridization reaction between the primer "C" and the nucleic acid complex from step (e) that has an exposed P̅3̅ domain for binding to the primer C. Step (g) illustrates the strand-displacing polymerization reaction initialized from the 3' end of P3 of the nucleic acid complex produced from step (f). Step (h) illustrates the resultant nucleic complexes produced from step (g). The released ssDNA 'U' functions as one of the essential LAMP primers for a downstream pre-designed highly optimized universal LAMP reaction.

Figure 2 illustrates three design schemes of the loaded primer A for use in the transduction reaction. The loaded primer A of each design scheme is shown inside the dashed box. Scheme (a) illustrates the input and output of the transduction reaction based on the first design of the loaded primer A. In this scheme, the loaded primer A consists of a primer nucleic acid strand with a single subsequence U̅ and a single subsequence P̅1̅ (listed from 5' to 3' orientation), loaded with a single nucleic acid strand U hybridized to the subsequence U̅. Upon detection of a single target strand, the reaction releases the ssDNA 'U' that functions as one of the essential LAMP primers for a downstream pre-designed optimized universal LAMP reaction. This corresponds to the transduction reaction as illustrated in Figure 1.

Scheme (b) illustrates the input and output of the transduction reaction based on a second design of the loaded primer A. In this scheme, the loaded primer A consists of a primer nucleic acid strand with multiple repeats of the subsequence U̅ and a single subsequence P̅1̅ (listed from 5' to 3' orientation), loaded with multiple copies of the nucleic acid strand U hybridized to multiple repeats of the subsequence U̅. Upon detection of a single target strand, the reaction releases multiple copies of the ssDNA 'U' to function as one of the essential LAMP primers for a downstream pre-designed optimized universal LAMP reaction. This design can be used to improve the assay sensitivity.

Scheme (c) illustrates the input and output of the transduction reaction based on a third design of the loaded primer A. In this scheme, the loaded primer A consists of a primer nucleic acid strand with a single subsequence U̅2̅, a single subsequence U̅1̅, and a single subsequence P̅1̅ (listed from 5' to 3' orientation), loaded with a single nucleic acid strand U2 and a single nucleic acid strand U1 hybridized to the subsequence U̅2̅ and the subsequence U̅1̅, respectively. Upon detection of a single target strand, the reaction co-releases the ssDNA 'U1' and ssDNA 'U2' that each function as separate essential LAMP primers for a downstream pre-designed optimized universal LAMP reaction.

In Figure 3, the components of the universal LAMP reaction are illustrated. Common reagents such as reaction buffer, nuclease-free water, and other standard amplification components known in the art are not depicted in the illustration but may be included. The universal LAMP reaction may include: (1) a pre-designed universal template for a highly optimized LAMP reaction, containing subsequences U̅, F2c, F1c, B1, B2, B3 (listed from 3' to 5' orientation) as six primer binding sites required for standard LAMP reactions; (2) the pre-optimized set of LAMP primers for rapid amplification of the universal template (1), including the F3 primer including a primer nucleic acid strand U, the B3 primer including a primer nucleic acid strand B3, the FIP primer including a primer nucleic acid strand with subsequences F1c and F2 (listed from 5' to 3' orientation), the BIP primer including a primer nucleic acid strand with subsequences B1c and B2 (listed from 5' to 3' orientation), the LoopF primer including a primer nucleic acid strand FLPc, and the LoopB primer including a primer nucleic acid strand BLPc; and (3) a strand-displacing polymerase. Similar to the bar notation, the 'c' modifier indicates sequence reverse complementarity. Thus, for example, F2c is reverse complementary to F2.

The present example utilizes the ssDNA 'U' released from the transduction reaction as the F3 primer in the universal LAMP reaction. However, depending on implementation preference, the ssDNA 'U' may be designed to serve as any one of the essential LAMP primers for optimal assay performance.

In Figure 4, the present invention's universal LAMP reaction is illustrated. The purpose of the universal LAMP reaction is to sense the release of the ssDNA 'U' from the transduction reaction to rapidly initialize a pre-designed and highly optimized LAMP reaction, the result of which can, for example, be easily interpreted by simple and rapid visual inspection of a pH-based colorimetric readout.

Step (a) illustrates the hybridization reaction between the universal template and the FIP primer via the primer binding site F2c on the universal template; step (b) illustrates the polymerization reaction initialized from the 3' end of F2 of the nucleic acid complex produced from step (a); step (c) illustrates the hybridization reaction between the ssDNA 'U' released from the transduction reaction and the nucleic acid complex produced from step (b) via the primer binding site U̅ on the universal template; step (d) illustrates the strand-displacing polymerization reaction initialized from the 3' end of U̅ of the nucleic acid complex produced from step (c), where the displaced ssDNA forms a nucleic acid complex with a loop structure at its 5' end; step (e) illustrates the hybridizations of the BIP primer and the B3 primer with the nucleic acid complex produced from step (d) via the primer binding site B2c and B3c on the complex, respectively; step (f) illustrates the strand-displacing polymerization reaction initialized from the 3' end of B2 of the nucleic acid complex produced from step (e) and the strand-displacing polymerization reaction initialized from the 3' end of B3 of the nucleic acid complex produced from step (e), where the displaced ssDNA forms a nucleic acid complex with the loop structure at both ends; step (g) illustrates the hybridizations of the FIP primer, the BIP primer, the LoopF primer, and the LoopB primer with the nucleic acid complex produced from step (f) via the primer binding site F2c, B2c, FLP, and BLP on the complex, respectively; step (h) indicates the LAMP's self-primed auto-cycling amplification facilitated by the strand-displacing polymerization reactions initialized from the 3' end of FIP, the 3' end of LoopB, the 3' end of BIP, and the 3' end of LoopF of the nucleic acid complex produced from step (g), respectively.

Figure 5 illustrates the reaction components of multiplexed universal LAMP supporting a 'Logic OR' operation in detection of multiple targets. The "Transduction Reaction Components" show input and output of the transduction reaction for detection of two distinct targets based on 'Logic OR'. The detection of either of the targets (or both) results in the release of a universal ssDNA oligo 'U' that initializes a downstream highly optimized universal LAMP reaction.

In the "Transduction Reaction Components," (1) illustrates the sequence composition of the first target strand, which contains subsequences P2, P1, P3 (listed from 3' to 5' orientation) as three adjacent primer binding sites, and (2) illustrates the three primers for detection of the first target strand, including: a loaded primer that includes a primer nucleic acid strand with subsequences U̅ and P̅1̅ (listed from 5' to 3' orientation), loaded with a nucleic acid strand U hybridized to the subsequence U̅; a primer that includes a primer nucleic acid strand P̅2̅; and a primer that includes a primer nucleic acid strand P3.

Also under "Transduction Reaction Components," (3) illustrates the sequence composition of the second target strand, which contains subsequences Q2, Q1, Q3 (listed from 3' to 5' orientation) as three adjacent primer binding sites, and (4) illustrates the three primers for detection of the second target strand, including: a loaded primer that includes a primer nucleic acid strand with subsequences U̅ and Q̅1̅ (listed from 5' to 3' orientation), loaded with a nucleic acid strand U hybridized to the subsequence U̅; a primer that includes a primer nucleic acid strand Q̅2̅; and a primer that includes a primer nucleic acid strand Q3. The detection of either the first target or the second target (or both) results in the release of the ssDNA oligonucleotide 'U' that functions as one of the essential LAMP primers for a downstream highly optimized universal LAMP reaction.

The "Universal LAMP Reaction Components" illustrates components of the universal LAMP reaction for detection of two distinct targets based on 'Logic OR': (1) is the sequence composition of a pre-designed universal template for a highly optimized LAMP reaction, containing subsequences U̅, F2c, F1c, B1, B2, B3 (listed from 3' to 5' orientation) as six primer binding sites required for standard LAMP reactions; (2) is the pre-optimized set of LAMP primers for rapid amplification of the universal template from (1), including the F3 primer including a primer nucleic acid strand U (provided by the transduction reaction), the B3 primer including primer nucleic acid strand B3, the FIP primer including a primer nucleic acid strand with subsequences F1c and F2 (listed from 5' to 3' orientation), the BIP primer including a primer nucleic acid strand with subsequences B1c and B2 (listed from 5' to 3' orientation), the LoopF primer including a primer nucleic acid strand FLPc, and the LoopB primer including a primer nucleic acid strand BLPc; and (3) is a strand-displacing polymerase.

Standard, known reagents such as reaction buffer and nuclease-free water are not depicted but may be included. In this example, the ssDNA 'U' released from the transduction reaction functions as the F3 primer in the universal LAMP reaction. Depending on the implementation preference, the ssDNA 'U' may be designed to serve as any one of the essential LAMP primers for optimal assay performance.

Figure 6 illustrates the reaction components of multiplexed universal LAMP supporting 'Logic AND' operation in detection of multiple targets. The "Transduction Reaction Components" illustrates the input and output of the transduction reaction for detection of two distinct targets based on 'Logic AND'. In particular, (1) illustrates the sequence composition of the first target strand, which contains subsequences P2, P1, P3 (listed from 3' to 5' orientation) as three adjacent primer binding sites, and (2) illustrates the three primers for detection of the first target strand, including: a loaded primer that includes a primer nucleic acid strand with subsequences U̅1̅ and P̅1̅ (listed from 5' to 3' orientation), loaded with a nucleic acid strand U1 hybridized to the subsequence U̅1̅; a primer that includes a primer nucleic acid strand P̅2̅; and a primer that includes a primer nucleic acid strand P3. The detection of the first target results in the release of an ssDNA oligo 'U1' (or multiple copies of 'U1' if using the multi-copy design scheme of the loaded primer) that functions as one of the essential LAMP primers for a downstream highly optimized universal LAMP reaction.

Also under "Transduction Reaction Components," (3) illustrates the sequence composition of the second target strand, which contains subsequences Q2, Q1, Q3 (listed from 3' to 5' orientation) as three adjacent primer binding sites, and (4) illustrates the three primers for detection of the second target strand, including: a loaded primer that includes a primer nucleic acid strand with subsequences U̅2̅ and Q̅1̅ (listed from 5' to 3' orientation), loaded with a nucleic acid strand U2 hybridized to the subsequence U̅2̅; a primer that includes a primer nucleic acid strand Q̅2̅; and a primer including nucleic acid strand Q3. The detection of the second target results in the release of a different ssDNA oligo 'U2' (or multiple copies of 'U2' if using the multi-copy design scheme of the loaded primer) that functions as another essential LAMP primer for the downstream LAMP reaction.

The "Universal LAMP Reaction Components" illustrates the components of the universal LAMP reaction for detection of two distinct targets based on 'Logic AND': (1) is the sequence composition of a pre-designed universal template for a highly optimized LAMP reaction. The template contains subsequences U̅1̅, F2c, F1c, B1, B2, U2 (listed from 3' to 5' orientation) as six primer binding sites required for standard LAMP reactions; (2) is the pre-optimized set of LAMP primers for rapid amplification of the universal template from (1), including the F3 primer including a primer nucleic acid strand U1 (provided by the transduction reaction), the B3 primer including a primer nucleic acid strand U2 (provided by the transduction reaction), the FIP primer including a primer nucleic acid strand with subsequences F1c and F2 (listed from 5' to 3' orientation), the BIP primer including a primer nucleic acid strand with subsequences B1c and B2 (listed from 5' to 3' orientation), the LoopF primer including a primer nucleic acid strand FLPc, and the LoopB primer including a primer nucleic acid strand BLPc; and (3) is a strand-displacing polymerase.

Standard, known reagents such as reaction buffer and nuclease-free water are not depicted but may be included. In this example, the ssDNA 'U1' and the ssDNA 'U2' released from the transduction reaction function as the F3 primer and the B3 primer for the downstream universal LAMP reaction, respectively. Depending on the implementation preference, the ssDNA 'U1' and 'U2' may be designed to serve as any pair of the essential LAMP primers for optimal assay performance.

Figure 7 illustrates a multiplexed universal LAMP reaction supporting 'Logic AND' operation in detection of multiple targets. The purpose of the universal LAMP reaction is to sense the co-release of the ssDNA 'U1' and the ssDNA 'U2' from the transduction reaction to rapidly initialize a pre-designed highly optimized LAMP reaction, the result of which can be, for example, easily interpreted by simple and rapid visual inspection of a pH-based colorimetric readout.

Step (a) illustrates the hybridization reaction between the universal template and the FIP primer via the primer binding site F2c on the universal template; step (b) illustrates the polymerization reaction initialized from the 3' end of F2 of the nucleic acid complex produced from step (a); step (c) illustrates the hybridization reaction between the ssDNA 'U1' released from the transduction reaction and the nucleic acid complex produced from step (b) via the primer binding site U̅1̅ on the universal template; step (d) illustrates the strand-displacing polymerization reaction initialized from the 3' end of U̅1̅ of the nucleic acid complex produced from step (c), where the displaced ssDNA forms a nucleic acid complex with a loop structure at its 5' end; step (e) illustrates the hybridizations of the BIP primer and the ssDNA 'U2' released from the transduction reaction with the nucleic acid complex produced from step (d) via the primer binding site B2c and U2c on the complex, respectively; step (f) illustrates the strand-displacing polymerization reaction initialized from the 3' end of B2 of the nucleic acid complex produced from step (e) and the strand-displacing polymerization reaction initialized from the 3' end of U2 of the nucleic acid complex produced from step (e), where the displaced ssDNA forms a nucleic acid complex with the loop structure at both ends; step (g) illustrates the hybridizations of the FIP primer, the BIP primer, the LoopF primer, and the LoopB primer with the nucleic acid complex produced from step (f) via the primer binding site F2c, B2c, FLP, and BLP on the complex, respectively; and step (h) indicates LAMP's self-primed auto-cycling amplification facilitated by the strand-displacing polymerization reactions initialized from the 3' end of FIP, the 3' end of LoopB, the 3' end of BIP, and the 3' end of LoopF of the nucleic acid complex produced from step (g), respectively.

The 'Logic OR' embodiment shown in Figure 5 and the 'Logic AND' embodiment shown in Figure 6 are examples illustrating how the transduction and LAMP reaction components may be configured to provide such logic operations, but the use of the present invention is not limited to these examples only. For example, for a 'Logic OR' operation, any number of additional primer subsets may be included, each directed to a different nucleic acid target, but each configured to generate the same LAMP primer in the presence of its respective target nucleic acid.

For a 'Logic AND' operation, up to six primer subsets may be included, each directed to a different nucleic acid target and each configured to generate a different LAMP primer in the presence of its respective target nucleic acid. Up to six of such primer subsets may be included because each of the potentially generated LAMP primers can correspond to one of the six LAMP primers of the universal LAMP template. Some embodiments may instead use up to four of such primer subsets, excluding the LoopF and LoopB primers so that they can instead be added directly to ensure their function of 'speeding up' the LAMP reaction is fully utilized.

Some embodiments may be configured for a 'Logic OR' determination in combination with a 'Logic AND' determination. For example, an embodiment may include a set of AND relationships with one or more nested OR relationships. As an example, a primer subset (a1) may be configured to generate a LAMP primer (A) in the presence of its nucleic acid target, and optionally one or more different primer subsets (a2, a3, a4, etc.) each also configured to generate the LAMP primer (A) in the presence of its respective nucleic acid target. Any number of additional (a)-type primer subsets may be included, each being configured to generate the LAMP primer (A) in the presence of its respective nucleic acid target. The embodiment can also include a primer subset (b1) configured to generate a different LAMP primer (B) in the presence of its nucleic acid target, and optionally one or more different primer subsets (b2, b3, b4, etc.) each also configured to generate the LAMP primer (B) in the presence of its respective nucleic acid target. Any number of additional (b)-type primer subsets may also be included, each being configured to generate the LAMP primer (B) in the presence of its respective nucleic acid target.

Such an embodiment incorporates AND logic because the LAMP reaction will only proceed if both the LAMP primer (A) and LAMP primer (B) are generated. The embodiment also incorporates OR logic because the LAMP primer (A) will be generated if at least one of the nucleic acid targets associated with the (a)-type primer subsets are present, and the LAMP (B) primer will be generated if at least one of the nucleic acid targets associated with the (b)-type primer subsets are present.

Some embodiments may incorporate additional AND relationships. For example, one or more (c)-type primer subsets may be included, each configured to generate a LAMP primer (C) in the presence of its respective nucleic acid target. The LAMP reaction will only proceed if each of LAMP primers (A), (B), and (C) are generated. As described above, up to six (or in some embodiments up to four) LAMP primers are generated in this manner, and each LAMP primer may optionally include multiple primer subsets with an OR relationship.

### Additional Terms & Definitions

While certain embodiments of the present disclosure have been described in detail, with reference to specific configurations, parameters, components, elements, etcetera, the descriptions are illustrative and are not to be construed as limiting the scope of the claimed invention.

Furthermore, it should be understood that for any given element of component of a described embodiment, any of the possible alternatives listed for that element or component may generally be used individually or in combination with one another, unless implicitly or explicitly stated otherwise.

In addition, unless otherwise indicated, numbers expressing quantities, constituents, distances, or other measurements used in the specification and claims are to be understood as optionally being modified by the term "about" or its synonyms. When the terms "about," "approximately," "substantially," or the like are used in conjunction with a stated amount, value, or condition, it may be taken to mean an amount, value or condition that deviates by less than 20%, less than 10%, less than 5%, less than 1%, less than 0.1%, or less than 0.01% of the stated amount, value, or condition. At the very least, and not as an attempt to limit the application of the doctrine of equivalents to the scope of the claims, each numerical parameter should be construed in light of the number of reported significant digits and by applying ordinary rounding techniques.

Any headings and subheadings used herein are for organizational purposes only and are not meant to be used to limit the scope of the description or the claims.

It will also be noted that, as used in this specification and the appended claims, the singular forms "a," "an" and "the" do not exclude plural referents unless the context clearly dictates otherwise. Thus, for example, an embodiment referencing a singular referent (e.g., "widget") may also include two or more such referents.

It will also be appreciated that embodiments described herein may include properties, features (e.g., ingredients, components, members, elements, parts, and/or portions) described in other embodiments described herein. Accordingly, the various features of a given embodiment can be combined with and/or incorporated into other embodiments of the present disclosure. Thus, disclosure of certain features relative to a specific embodiment of the present disclosure should not be construed as limiting application or inclusion of said features to the specific embodiment. Rather, it will be appreciated that other embodiments can also include such features.

## Claims

1. A method of performing loop-mediated isothermal amplification (LAMP) of a target nucleic acid, the method comprising:
non-surgically providing a sample; and
performing a transduction reaction, the transduction reaction functioning to generate a single stranded DNA (ssDNA) oligonucleotide when the target nucleic acid is present in the sample;
wherein the ssDNA generated by the transduction reaction functions as a required LAMP primer for a universal LAMP template, the LAMP primer thereby, when present, enabling a LAMP reaction to proceed.

2. The method of claim 1, wherein the LAMP reaction is a reverse transcription LAMP (RT-LAMP) reaction.

3. The method of claim 1 or 2, wherein the LAMP template is independent of the target nucleic acid.

4. The method of any one of claims 1 to 3, wherein other required LAMP primers are provided such that the presence of the LAMP primer resulting from the transduction reaction determines whether the LAMP reaction will proceed.

5. The method of any one of claims 1 to 4, wherein the transduction reaction comprises:
mixing a set of transduction primers to the sample, the transduction primers being configured to associate with the target nucleic acid;
wherein a first transduction primer (A) includes:
a subsequence P̅1̅ capable of hybridizing with a subsequence P1 of the target nucleic acid,
a subsequence U̅, and
a nucleic acid strand U hybridized to the subsequence U̅, wherein nucleic acid strand U is the LAMP primer;
wherein a second transduction primer (B) includes a sequence P̅2̅ capable of hybridizing with a subsequence P2 of the target nucleic acid;
wherein a third transduction primer (C) includes a sequence P3 matching a P3 subsequence of the target nucleic acid; and
wherein amplification using the set of transduction primers and a strand-displacing polymerase results in release of U.

6. The method of claim 5, wherein P2 is 3' of P1, and wherein P3 is 5' of P1 on the target nucleic acid.

7. The method of claim 5 or 6, wherein U̅ is 5' of P̅1̅ in the first transduction primer (A).

8. The method of any one of claims 1 to 7, wherein the transduction reaction is configured as an "OR" reaction, the transduction reaction functioning to generate the same transduced LAMP primer when any one of two or more target nucleic acids are present in the sample or wherein the transduction reaction is configured as an "AND" reaction, the transduction reaction functioning to generate a different required LAMP primer for each targeted nucleic acid present within the sample such that the LAMP reaction proceeds only if each targeted nucleic acid is present within the sample.

9. The method of any one of claims 1 to 8, wherein the target nucleic acid is associated with an infectious disease, preferably the infectious disease is Ebola, Zika, COVID-19, flu, malaria, tuberculosis, dengue, or yellow fever.

10. The method of any one of claims 1 to 9, wherein the sample is an environmental sample, preferably a water sample or a soil sample, and wherein the method is performed as part of an environmental monitoring or environmental assessment process.

11. A composition formulated to enable loop-mediated isothermal amplification (LAMP) of a target nucleic acid, the composition comprising:
a universal LAMP template;
an incomplete set of LAMP primers corresponding to the universal LAMP template; and
a set of primers configured to associate with the target nucleic acid, wherein the set of primers is configured to enable a transduction reaction that generates a single stranded DNA (ssDNA) oligonucleotide when the target nucleic acid is present in the sample, the ssDNA functioning as one of the LAMP primers to promote a LAMP reaction.

12. The composition of claim 11, further comprising a strand-displacing DNA polymerase, and optionally, a reverse transcriptase.

13. The composition of claim 11 or 12, wherein the LAMP template is independent of the target nucleic acid.

14. The composition of any one of claims 11 to 13, wherein the set of primers includes at least two primer subsets each directed to a different nucleic acid target.

15. The composition of claim 14, wherein the set of primers is configured for a multi-input 'Logic OR' determination, wherein two or more primer subsets are each configured to generate the same LAMP primer in the presence of their respective nucleic acid targets to thereby enable the LAMP reaction to proceed in the presence of any of these nucleic acid targets, preferably the incomplete set of LAMP primers lacks only the LAMP primer generated in the presence of the nucleic acid targets of the two or more primer subsets such that the LAMP reaction can proceed in the presence of any of the nucleic acid targets of the two or more primer subsets.

16. The composition of claim 14 or 15, wherein the set of primers is configured for a multi-input 'Logic AND' determination, wherein two or more primer subsets, optionally up to six subsets, are each configured to generate a different LAMP primer in the presence of their respective nucleic acid targets to thereby enable the LAMP reaction to proceed only in the presence of each of these nucleic acid targets, preferably the incomplete set of LAMP primers lacks each of the LAMP primers generated in the presence of the nucleic acid targets of the two or more primer subsets such that the LAMP reaction can only proceed in the presence of each of the nucleic acid targets of the two or more primer subsets.

17. The composition of any one of claims 14 to 16, wherein the set of primers is configured for a 'Logic OR' determination in combination with a 'Logic AND' determination, the set of primers comprising:
one or more primer subsets (a) each configured to generate the same LAMP primer (A) in the presence of their respective nucleic acid targets such that the LAMP primer (A) is generated in the presence of any of the nucleic acid targets of the one or more primer subsets (a); and two or more primer subsets (b) each configured to generate the same LAMP primer (B) in the presence of their respective nucleic acid targets such that the LAMP primer (B) is generated in the presence of any of the nucleic acid targets of the two or more primer subsets (b);
wherein the LAMP reaction proceeds only where both LAMP primer (A) and LAMP primer (B) are generated, optionally the set of primers comprises two or more primer subsets (a) each configured to generate the same LAMP primer (A) in the presence of their respective nucleic acid targets.

18. The composition of claim 17, further comprising one or more primer subsets, in addition to primer subsets (a) and primer subsets (b), configured to generate a LAMP primer different from LAMP primer (A) and LAMP primer (B) in the presence of their respective nucleic acid targets, wherein the LAMP reaction proceeds only where all of LAMP primer (A), LAMP primer (B), and the one or more additional LAMP primers are generated.

## Patentansprüche

1. Verfahren zur Durchführung einer Schleifen-vermittelten isothermen Amplifikation (LAMP) einer Zielnukleinsäure, wobei das Verfahren umfasst:
das nicht-chirurgische Bereitstellen einer Probe; und
die Durchführung einer Transduktionsreaktion, wobei die Transduktionsreaktion dazu dient, ein einzelsträngiges DNA-Oligonukleotid (ssDNA) zu erzeugen, wenn die Zielnukleinsäure in der Probe vorhanden ist;
wobei die durch die Transduktionsreaktion erzeugte ssDNA als erforderlicher LAMP-Primer für eine universelle LAMP-Matrize fungiert, wobei der LAMP-Primer, sofern vorhanden, den Ablauf einer LAMP-Reaktion ermöglicht.

2. Verfahren nach Anspruch 1, wobei die LAMP-Reaktion eine Reverse-Transkriptions-LAMP-Reaktion (RT-LAMP) ist.

3. Verfahren nach Anspruch 1 oder 2, wobei die LAMP-Matrize unabhängig von der Zielnukleinsäure ist.

4. Verfahren nach einem der Ansprüche 1 bis 3, wobei andere erforderliche LAMP-Primer bereitgestellt werden, so dass das Vorhandensein des aus der Transduktionsreaktion resultierenden LAMP-Primers bestimmt, ob die LAMP-Reaktion abläuft.

5. Verfahren nach einem der Ansprüche 1 bis 4, wobei die Transduktionsreaktion umfasst:
das Mischen eines Satzes von Transduktionsprimern mit der Probe, wobei die Transduktionsprimer so konfiguriert sind, dass sie sich an die Zielnukleinsäure binden;
wobei ein erster Transduktionsprimer (A) umfasst:
eine Teilsequenz P̅1̅, die in der Lage ist, mit einer Teilsequenz P1 der Zielnukleinsäure zu hybridisieren,
eine Teilsequenz U̅, und
einen Nukleinsäurestrang U, der an die Teilsequenz U̅ hybridisiert ist, wobei der Nukleinsäurestrang U der LAMP-Primer ist;
wobei ein zweiter Transduktionsprimer (B) eine Sequenz P̅2̅ umfasst, die in der Lage ist, mit einer Teilsequenz P2 der Zielnukleinsäure zu hybridisieren;
wobei ein dritter Transduktionsprimer (C) eine Sequenz P3 enthält, die mit einer P3-Teilsequenz der Zielnukleinsäure übereinstimmt; und
wobei die Amplifikation unter Verwendung des Satzes von Transduktionsprimern und einer strangverdrängenden Polymerase zur Freisetzung von U führt.

6. Verfahren nach Anspruch 5, wobei P2 3'-seitig von P1 liegt und wobei P3 5'-seitig von P1 auf der Zielnukleinsäure liegt.

7. Verfahren nach Anspruch 5 oder 6, wobei "U̅ " 5'-seitig von "P̅1̅ " im ersten Transduktionsprimer (A) liegt.

8. Verfahren nach einem der Ansprüche 1 bis 7, wobei die Transduktionsreaktion als "ODER"-Reaktion konfiguriert ist, wobei die Transduktionsreaktion so funktioniert, dass derselbe transduzierte LAMP-Primer erzeugt wird, wenn eine von zwei oder mehr Zielnukleinsäuren in der Probe vorhanden ist, oder wobei die Transduktionsreaktion als "UND"-Reaktion konfiguriert ist, wobei die Transduktionsreaktion so funktioniert, dass für jede in der Probe vorhandene Zielnukleinsäure ein anderer erforderlicher LAMP-Primer erzeugt wird, sodass die LAMP-Reaktion nur dann abläuft, wenn jede Zielnukleinsäure in der Probe vorhanden ist.

9. Verfahren nach einem der Ansprüche 1 bis 8, wobei die Zielnukleinsäure mit einer Infektionskrankheit assoziiert ist, wobei vorzugsweise die Infektionskrankheit Ebola, Zika, COVID-19, Grippe, Malaria, Tuberkulose, Dengue-Fieber oder Gelbfieber ist.

10. Verfahren nach einem der Ansprüche 1 bis 9, wobei die Probe eine Umweltprobe ist, vorzugsweise eine Wasserprobe oder eine Bodenprobe, und wobei das Verfahren als Teil eines Umweltüberwachungs- oder Umweltbewertungsprozesses durchgeführt wird.

11. Zusammensetzung, die so formuliert ist, dass sie eine Schleifen-vermittelte isotherme Amplifikation (LAMP) einer Zielnukleinsäure ermöglicht, wobei die Zusammensetzung umfasst:
eine universelle LAMP-Matrize;
einen unvollständigen Satz von LAMP-Primern, die der universellen LAMP-Matrize entsprechen; und
ein Satz von Primern, der so konfiguriert ist, dass er an die Zielnukleinsäure bindet, wobei der Satz von Primern so konfiguriert ist, dass er eine Transduktionsreaktion ermöglicht, bei der ein einzelsträngiges DNA-Oligonukleotid (ssDNA) entsteht, wenn die Zielnukleinsäure in der Probe vorhanden ist, wobei die ssDNA als einer der LAMP-Primer fungiert, um eine LAMP-Reaktion zu fördern.

12. Zusammensetzung nach Anspruch 11, ferner umfassend eine strangverdrängende DNA-Polymerase und optional eine Reverse Transkriptase.

13. Die Zusammensetzung nach Anspruch 11 oder 12, wobei die LAMP-Matrize unabhängig von der Zielnukleinsäure ist.

14. Zusammensetzung nach einem der Ansprüche 11 bis 13, wobei der Satz von Primern mindestens zwei Teilsätze von Primern umfasst, die jeweils auf eine andere Nukleinsäure-Zielsequenz gerichtet sind.

15. Die Zusammensetzung nach Anspruch 14, wobei der Satz von Primern für eine Multi-Input-"Logik ODER"-Bestimmung konfiguriert ist, wobei zwei oder mehr Teilsätze von Primern jeweils so konfiguriert sind, dass sie in Gegenwart ihrer jeweiligen Nukleinsäure-Zielsequenzen denselben LAMP-Primer erzeugen, um dadurch den Ablauf der LAMP-Reaktion in Gegenwart einer beliebigen dieser Nukleinsäure-Zielsequenzen zu ermöglichen, vorzugsweise fehlt in dem unvollständigen Satz von LAMP-Primern nur der LAMP-Primer, der in Gegenwart der Nukleinsäure-Zielsequenzen der zwei oder mehr Teilsätzen von Primern erzeugt wird, so dass die LAMP-Reaktion in Gegenwart von jeglicher Nukleinsäure-Zielsequenzen der zwei oder mehr Teilsätzen von Primern ablaufen kann.

16. Die Zusammensetzung nach Anspruch 14 oder 15, wobei der Satz von Primern für eine Multi-Input-"Logik-UND"-Bestimmung konfiguriert ist, wobei zwei oder mehr Teilsätze von Primern, optional bis zu sechs Teilsätzen, jeweils so konfiguriert sind, dass sie in Gegenwart ihrer jeweiligen Nukleinsäure-Zielsequenzen einen unterschiedlichen LAMP-Primer erzeugen, um dadurch zu ermöglichen, dass die LAMP-Reaktion nur in Gegenwart jeder dieser Nukleinsäure-Zielsequenzen abläuft, vorzugsweise fehlt dem unvollständigen Satz von LAMP-Primern jeder der LAMP-Primer, die in Gegenwart der Nukleinsäure-Zielsequenzen der zwei oder mehr Teilsätze von Primern erzeugt werden, so dass die LAMP-Reaktion nur in Gegenwart jeder der Nukleinsäure-Zielsequenzen der zwei oder mehr Teilsätze von Primern ablaufen kann.

17. Die Zusammensetzung nach einem der Ansprüche 14 bis 16, wobei der Satz von Primern für eine "Logik-ODER"-Bestimmung in Kombination mit einer "Logik-UND"-Bestimmung konfiguriert ist, wobei der Satz von Primern umfasst:
eine oder mehrere Teilsätze von Primern (a), die jeweils so konfiguriert sind, dass sie in Gegenwart ihrer jeweiligen Nukleinsäure-Zielsequenzen denselben LAMP-Primer (A) erzeugen, so dass der LAMP-Primer (A) in Gegenwart jeglicher Nukleinsäure-Zielsequenzen der einen oder mehreren Teilsätzen von Primern (a) erzeugt wird; und zwei oder mehr Teilsätze von Primern (b), die jeweils so konfiguriert sind, dass sie in Gegenwart ihrer jeweiligen Nukleinsäure-Zielsequenzen denselben LAMP-Primer (B) erzeugen, sodass der LAMP-Primer (B) in Gegenwart jeglicher Nukleinsäure-Zielsequenzen der zwei oder mehr Primerverzweigungen (b) erzeugt wird;
wobei die LAMP-Reaktion nur dann abläuft, wenn sowohl der LAMP-Primer (A) als auch der LAMP-Primer (B) erzeugt werden, wobei der Satz von Primern optional zwei oder mehr Teilsätze von Primern (a) umfasst, die jeweils so konfiguriert sind, dass sie denselben LAMP-Primer (A) in Gegenwart ihrer jeweiligen Nukleinsäure-Zielsequenzen erzeugen.

18. Die Zusammensetzung nach Anspruch 17, die zusätzlich zu den Teilsätzen von Primern (a) und den Teilsätzen von Primern (b) eine oder mehrere Teilsätze von Primern umfasst, die so konfiguriert sind, dass sie in Gegenwart ihrer jeweiligen Nukleinsäure-Zielsequenzen einen LAMP-Primer erzeugen, der sich von dem LAMP-Primer (A) und dem LAMP-Primer (B) unterscheidet, wobei die LAMP-Reaktion nur dann abläuft, wenn alle von LAMP-Primer (A), LAMP-Primer (B) und dem einen oder mehreren zusätzlichen LAMP-Primer erzeugt werden.

## Revendications

1. Procédé de réalisation d'une amplification isotherme médiée par boucle (LAMP) d'un acide nucléique cible, le procédé comprenant:
fournir un échantillon de manière non chirurgicale; et
réaliser une réaction de transduction, la réaction de transduction fonctionnant pour générer un oligonucléotide d'ADN simple brin (ADNsb) lorsque l'acide nucléique cible est présent dans l'échantillon;
l'ADNsb généré par la réaction de transduction fonctionnant comme une amorce LAMP requise pour une matrice LAMP universelle, l'amorce LAMP permettant ainsi, lorsqu'elle est présente, le déroulement d'une réaction LAMP.

2. Procédé selon la revendication 1, dans lequel la réaction LAMP est une réaction de transcription inverse LAMP (RT-LAMP).

3. Procédé selon la revendication 1 ou 2, dans lequel la matrice LAMP est indépendante de l'acide nucléique cible.

4. Procédé selon l'une quelconque des revendications 1 à 3, dans lequel d'autres amorces LAMP requises sont fournies de telle sorte que la présence de l'amorce LAMP résultant de la réaction de transduction détermine si la réaction LAMP se déroulera.

5. Procédé selon l'une quelconque des revendications 1 à 4, dans lequel la réaction de transduction comprend:
mélanger un ensemble d'amorces de transduction à l'échantillon, les amorces de transduction étant configurées pour s'associer à l'acide nucléique cible;
dans lequel une première amorce de transduction (A) comprend:
une sous-séquence P̅1̅ capable de s'hybrider avec une sous-séquence P1 de l'acide nucléique cible,
une sous-séquence U̅, et
un brin d'acide nucléique U hybridé à la sous-séquence U̅, le brin d'acide nucléique U étant l'amorce LAMP;
dans lequel une deuxième amorce de transduction (B) comprend une séquence P̅2̅ capable de s'hybrider avec une sous-séquence P2 de l'acide nucléique cible;
dans lequel une troisième amorce de transduction (C) comprend une séquence P3 correspondant à une sous-séquence P3 de l'acide nucléique cible; et
dans lequel l'amplification à l'aide de l'ensemble d'amorces de transduction et d'une polymérase à déplacement de brin résulte en la libération de U.

6. Procédé selon la revendication 5, dans lequel P2 est situé en 3' de P1, et dans lequel P3 est situé en 5' de P1 sur l'acide nucléique cible.

7. Procédé selon la revendication 5 ou 6, dans lequel U̅ est situé en 5' de P̅1̅ dans la première amorce de transduction (A).

8. Procédé selon l'une quelconque des revendications 1 à 7, dans lequel la réaction de transduction est configurée comme une réaction "OU", la réaction de transduction fonctionnant pour générer la même amorce LAMP transduite lorsque l'un quelconque des deux ou plusieurs acides nucléiques cibles est présent dans l'échantillon, ou dans lequel la réaction de transduction est configurée comme une réaction "ET", la réaction de transduction fonctionnant pour générer une amorce LAMP requise différente pour chaque acide nucléique ciblé présent dans l'échantillon, de telle sorte que la réaction LAMP ne se déroule que si chaque acide nucléique ciblé est présent dans l'échantillon.

9. Procédé selon l'une quelconque des revendications 1 à 8, dans lequel l'acide nucléique cible est associé à une maladie infectieuse, la maladie infectieuse étant, de préférence, l'Ebola, le Zika, le COVID-19, la grippe, le paludisme, la tuberculose, la dengue ou la fièvre jaune.

10. Procédé selon l'une quelconque des revendications 1 à 9, dans lequel l'échantillon est un échantillon environnemental, de préférence un échantillon d'eau ou un échantillon de sol, et dans lequel le procédé est réalisé dans le cadre d'un processus de surveillance environnementale ou d'évaluation environnementale.

11. Composition formulée pour permettre l'amplification isotherme médiée par boucle (LAMP) d'un acide nucléique cible, la composition comprenant:
une matrice LAMP universelle;
un ensemble incomplet d'amorces LAMP correspondant à la matrice LAMP universelle; et
un ensemble d'amorces configurées pour s'associer à l'acide nucléique cible, l'ensemble d'amorces étant configuré pour permettre une réaction de transduction générant un oligonucléotide d'ADN simple brin (ADNsb) lorsque l'acide nucléique cible est présent dans l'échantillon, l'ADNsb fonctionnant comme l'une des amorces LAMP pour promouvoir une réaction LAMP.

12. Composition selon la revendication 11, comprenant en outre une ADN polymérase à déplacement de brin et, éventuellement, une transcriptase inverse.

13. Composition selon la revendication 11 ou 12, dans laquelle la matrice LAMP est indépendante de l'acide nucléique cible.

14. Composition selon l'une quelconque des revendications 11 à 13, dans laquelle l'ensemble d'amorces comprend au moins deux sous-ensembles d'amorces, chacun dirigé vers une cible d'acide nucléique différente.

15. Composition selon la revendication 14, dans laquelle l'ensemble d'amorces est configuré pour une détermination "logique OU" à entrées multiples, dans laquelle deux ou plusieurs sous-ensembles d'amorces étant chacun configurés pour générer la même amorce LAMP en présence de leurs cibles d'acide nucléique respectives, permettant ainsi à la réaction LAMP de se dérouler en présence de n'importe laquelle de ces cibles d'acide nucléique, de préférence l'ensemble incomplet d'amorces LAMP ne manquant que de l'amorce LAMP générée en présence des cibles d'acide nucléique des deux ou plusieurs sous-ensembles d'amorces, de telle sorte que la réaction LAMP puisse se dérouler en présence de n'importe laquelle des cibles d'acide nucléique des deux ou plusieurs sous-ensembles d'amorces.

16. Composition selon la revendication 14 ou 15, dans laquelle l'ensemble d'amorces est configuré pour une détermination "logique ET" à entrées multiples, dans laquelle deux ou plusieurs sous-ensembles d'amorces, éventuellement jusqu'à six sous-ensembles, étant chacun configurés pour générer une amorce LAMP différente en présence de leurs cibles d'acide nucléique respectives, permettant ainsi à la réaction LAMP de ne se dérouler qu'en présence de chacune de ces cibles d'acide nucléique, de préférence l'ensemble incomplet d'amorces LAMP manquant de chacune des amorces LAMP générées en présence des cibles d'acide nucléique des deux ou plusieurs sous-ensembles d'amorces, de telle sorte que la réaction LAMP ne puisse se dérouler qu'en présence de chacune des cibles d'acide nucléique des deux ou plusieurs sous-ensembles d'amorces.

17. Composition selon l'une quelconque des revendications 14 à 16, dans laquelle l'ensemble d'amorces est configuré pour une détermination "logique OU" en combinaison avec une détermination "logique ET", l'ensemble d'amorces comprenant:
un ou plusieurs sous-ensembles d'amorces (a) configurés chacun pour générer la même amorce LAMP (A) en présence de leurs cibles d'acide nucléique respectives, de telle sorte que l'amorce LAMP (A) soit générée en présence de n'importe laquelle des cibles d'acide nucléique d'un ou des plusieurs sous-ensembles d'amorces (a); et deux ou plusieurs sous-ensembles d'amorces (b) configurés chacun pour générer la même amorce LAMP (B) en présence de leurs cibles d'acide nucléique respectives, de telle sorte que l'amorce LAMP (B) soit générée en présence de n'importe laquelle des cibles d'acide nucléique des deux ou plusieurs sous-ensembles d'amorces (b);
dans laquelle la réaction LAMP ne se déroule que lorsque l'amorce LAMP (A) et l'amorce LAMP (B) sont toutes deux générées, l'ensemble d'amorces comprenant éventuellement deux ou plusieurs sous-ensembles d'amorces (a), chacun configuré pour générer la même amorce LAMP (A) en présence de leurs cibles d'acide nucléique respectives.

18. Composition selon la revendication 17, comprenant en outre un ou plusieurs sous-ensembles d'amorces, en plus des sous-ensembles d'amorces (a) et des sous-ensembles d'amorces (b), configurés pour générer une amorce LAMP différente de l'amorce LAMP (A) et de l'amorce LAMP (B) en présence de leurs cibles d'acide nucléique respectives, dans laquelle la réaction LAMP ne se déroule que lorsque l'amorce LAMP (A), l'amorce LAMP (B) et l'une ou les plusieurs amorces LAMP supplémentaires sont toutes générées.
